# EUROPÄISCHE PATENTSCHRIFT

(11) **EP 1 219 182 B1**
(45) Veröffentlichungstag und Bekanntmachung des Hinweises auf die Patenterteilung: **09.11.2005**
(21) Anmeldenummer: 01129349.5
(22) Anmeldetag: 17.12.2001
(51) Int. Cl.: A23L 1/305, A23L 1/236

(54) **Salze von L-Aminosäuren mit verbessertem Geschmack und ihre Herstellung**
Better tasting L-amino acid salts and their manufacture
Sels d'acides L-aminés ayant un goût amélioré et leur préparation

(30) Priorität: 27.12.2000 US 749136
(43) Veröffentlichungstag der Anmeldung: 03.07.2002
(73) Patentinhaber: Nutrinova Nutrition Specialties & Food Ingredients GmbH, 65929 Frankfurt am Main (DE)
(72) Erfinder: Burgard, Andreas, Dr., 65929 Frankfurt am Main (DE); Roy, Glenn, Beacon, New York 12508 (US)
(74) Vertreter: Schweitzer, Klaus

(56) Entgegenhaltungen:
- EP-A- 0 122 400
- EP-A- 1 013 633
- WO-A-99/04822
- PATENT ABSTRACTS OF JAPAN vol. 1996, no. 09, 30. September 1996 (1996-09-30) & JP 08 134034 A (AJINOMOTO CO INC), 28. Mai 1996 (1996-05-28)

## Beschreibung

Die Aminosäuren Arginin und Lysin werden in der Regel zu den essentiellen Aminosäuren gezählt. Es kann aus ernährungsphysiologischer Sicht wünschenswert sein, Lebensmittel mit diesen Aminosäuren anzureichern. Insbesondere werden für Arginin aber auch Anwendungen im therapeutischen Bereich diskutiert, z. B. zur Behandlung von Hypertonie und anderen Blutgefäßerkrankungen (EP-A 0 441 119), als Arzneimittel bei Diabetes mellitus (EP-A 0 370 994) und als Medikament zur Behandlung von Infektionen mit Helicobacter Pylori-Bakterien (WO 98/57626).

Der Geschmack dieser Aminosäure ist allerdings nicht besonders angenehm. Besonders Arginin ist deutlich bitter, was seiner direkten Verwendung in Zubereitungen zur oralen Aufnahme Grenzen setzt. Deshalb muß bisher der Geschmack von Arginin in solchen Präparaten aufwendig durch Geschmacksstoffe maskiert werden und auch bei Lysin ist eine geschmackliche Verbesserung mit Geschmacksstoffen angezeigt.

In DE-A 1 242 622, EP-A 0 046 506, WO-A 99/04822 und WO-A 00/12067 werden Süßstoff-Medikament-Salze mit verbessertem Geschmack beschrieben, wobei jeweils der Süßstoff als Anion und das Medikament als Monokation im Verhältnis 1:1 vorliegt. Diese Anmeldungen beschreiben jedoch nicht die Geschmacksverbesserung bzw. Geschmacksmaskierung von bitter schmeckenden Aminosäuren. Insbesondere sind Salze zweibasiger Aminosäuren wie z.B. Arginin, Lysin und Ornithin, - die nicht als Medikament, sondern eher als essentielle Aminosäuren zu sehen sind -, dort nicht beschrieben.

Die EP-A-0122 400 beschreibt Acesulfam-K-Mischungen, die den bitteren Geschmack von Acesulfam-K neutralisieren sollen. Hierzu wird Acesulfam-K mit einem oder mehreren Additiven versetzt, die aus einer Liste ausgewählt sind, die Weinsäure, diverse Aminosäuren und Natriumphosphat enthält.

Es wurde nun gefunden, dass der Süßstoff Acesulfam, der in Form seines Kaliumsalzes [6-Methyl-3,4-dihydro-1,2,3-oxathiazin-4-on-2,2-dioxid-Kaliumsalz] in großem Umfang in Lebensmitteln, Mundkosmetika und Arzneimitteln eingesetzt wird, mit basisch reagierenden Aminosäuren salzartige Verbindungen bilden kann, die überraschenderweise nicht mehr bitter, sondern rein süß schmecken. Damit wird die Verwendung dieser Aminosäuren, insbesondere von Arginin in Präparaten zur oralen Verwendung deutlich vereinfacht.

Die Herstellung dieser salzartigen Verbindungen ist einfach. Beispielsweise wird ein Äquivalent L-Arginin (1) mit einem Äquivalent Acesulfam-H (2) in Wasser zur Reaktion gebracht, wobei L-Arginin als Base und Acesulfam-H als Säure fungieren. Acesulfam-H ist die korrespondierende Säure zu dem kommerziell erhältlichen Acesulfam-K (z.B. Sunett®, Nutrinova, Frankfurt a.M., Deutschland), welches durch Protonierung mittels einer starken Säure wie z.B. Schwefelsäure zu Acesulfam-H umgesetzt werden kann. Acesulfam-H und Acesulfam-K können auch nach Literatur bekannten Methoden dargestellt werden (vgl. EP-A 0 155 634).

Die verwendeten Aminosäuren sind kommerziell erhältlich.

Das resultierende Salzaddukt (3) ist außerdem wesentlich besser in Wasser löslich als das nicht protonierte, freie L-Arginin (1). Aus der so erhaltenen Reaktionslösung wird das Reaktionsprodukt in einfacher Weise durch Entfernen des Wassers gewonnen z. B. durch Verdampfen im Vakuum. Das L-Arginin-Acesulfam-salz (3) liegt laut ¹H-NMR als 1:1-Addukt vor.

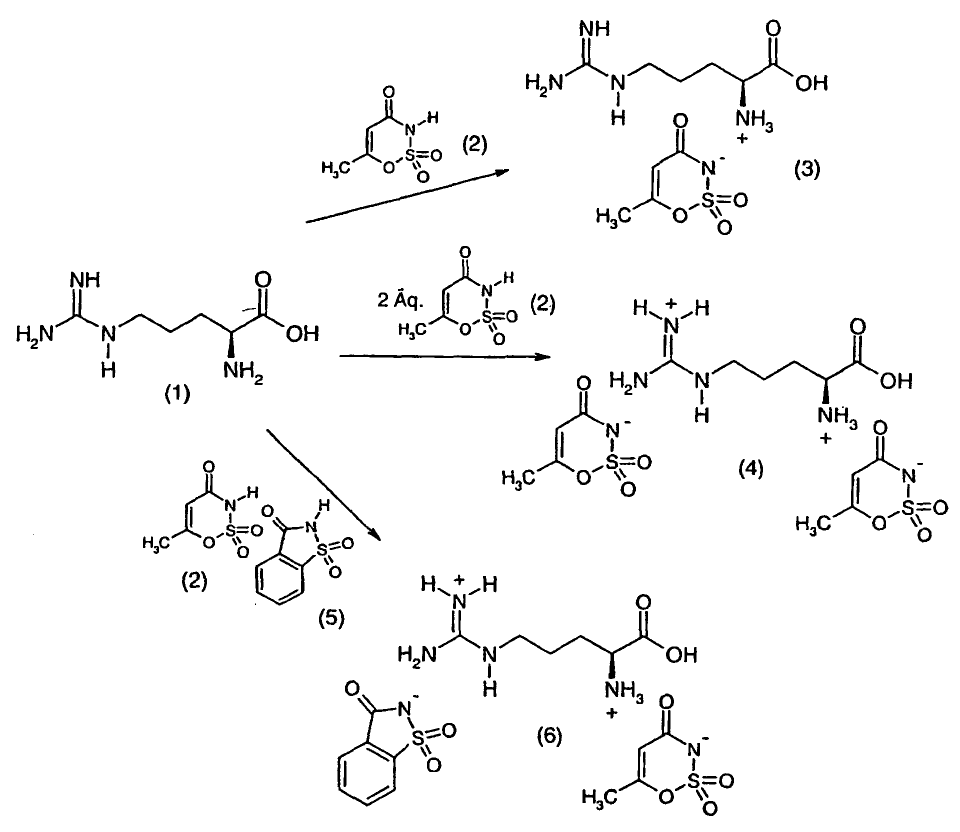

Werden zwei Äquivalente Acesulfam-H (2) als Säure eingesetzt, so resultiert ein ebenfalls stöchiometrisches 1:2-Addukt eines L-Arginin-Acesulfamsalzes (4), dessen Struktur mittels ¹H-NMR bestätigt werden konnte. Sowohl das 1:1 als auch das 1:2-L-Arginin-Acesulfam-Addukt haben einen angenehm süßen Geschmack ohne den bitteren Geschmack des L-Arginins. Die Intensität der Süße je Gewichtseinheit liegt beim 1:2 Addukt dabei deutlich über derjenigen des 1:1 Adduktes.

Die Umsetzung von L-Arginin mit einem Äquivalent Acesulfam-H (2) und einem Äquivalent Saccharin-H (5) als Säure, welche analog zum Acesulfam-H die korrespondierende Säure zum kommerziell genutzten Süßstoff Saccharin-Natrium darstellt, ergab ein ebenfalls süß schmeckendes 1:1:1-L-Arginin-Acesulfam-Saccharin-Addukt (6), welches durch ¹H-NMR-Spektroskopie bestätigt werden konnte.

In gleicher Weise können mit allen Anionen bildenden Süßstoffen wie z.B. Acesulfam, Saccharin, Aspartam, Neotam, Alitam, Glycyrrhizin und Gluconsäure mit basisch reagierenden Aminosäuren entsprechende Salze (Addukte) hergestellt werden. Dabei ist eine Vielzahl von Kombinationen, insbesondere bei den 1:2-Addukten möglich, die sich in ihren geschmacklichen Eigenschaften, insbesondere im zeitlichen Verlauf der Süße und der Süßeintensität, deutlich voneinander unterscheiden. Das gilt insbesondere für Addukte aus einem Molekül Arginin und zwei Molekülen Süßstoff, bei denen 1:2-Addukte von L-Arginin mit dem gleichen Süßstoff oder 1:1:1-Addukte mit verschiedenen Süßstoffen hergestellt werden, von denen die letzteren eine besonders große Zahl geschmacklicher Varianten ermöglichen. Die Umsetzung von L-Arginin mit einem Äquivalent Acesulfam-H und einem Äquivalent Saccharin-H als Säure ergibt beispielsweise ein ebenfalls süß schmeckendes 1:1:1-L-Arginin-Acesulfam-Saccharin-Addukt.

Eine Variante einer oben benannten Herstellung der erfindungsgemäßen Addukte besteht darin, dass man die Süßstoffe in Form ihrer physiologisch verträglichen Salze einsetzt und die Reaktion in Gegenwart einer physiologisch verträglichen Säure, die als Protonenquelle dient, durchführt. Verwendbare physiologisch verträgliche Säuren sind bspw. Salzsäure, Schwefelsäure, Phosphorsäure, Essigsäure; bevorzugt wird Salzsäure verwendet.

Die Methode, den Geschmack basisch reagierender Aminosäuren durch eine Salzbildung mit Anionen bildenden Süßstoffen zu maskieren, ist nicht nur auf L-Arginin beschränkt, sondern kann auch allgemein auf andere vergleichbar reagierende Aminosäuren angewendet werden, insbesondere auf das L-Ornithin, L-Histidin, L-Tryptophan und das L-Lysin.

Die oben angeführten Aminosäure-Süßstoff-Addukte sind wasserlöslich und können in kristalliner Form hergestellt und als solche in Präparate zur oralen Verwendung wie z.B. Tabletten und Komprimaten der verschiedenen Art, Kaugummi und Kautabletten eingearbeitet werden. Sie haben außerdem den Vorteil, dass sie sich als Salze nicht entmischen, was geschmackliche Inhomogenitäten zur Folge hätte. Solche Entmischungen sind ein in der Lebensmittel- und Arzneimittelherstellung bekanntes Problem.

Auf Grund ihrer Wasserlöslichkeit eignen sie sich ebenso für die Verwendung in flüssigen Produkten wie Getränken oder Sirupen oder zum Einsatz in festen, zur Auflösung bestimmten Präparaten wie Getränkepulvern oder Brausetabletten.

Die Erfindung wird durch die folgenden Beispiele erläutert:

### Beispiel 1

### Herstellung des 1:1-L-Arginin-Acesulfam-Adduktes

In 20 ml Wasser werden 15 mmol (2,613 g) L-Arginin und 15 mmol (2,447 g) Acesulfam-H gelöst. Das Reaktionsgemisch wird anschließend im Vakuum eingeengt. Es resultieren mit 100 % Ausbeute farblose Kristalle, die laut ¹H-NMR als 1:1-Addukt vorliegen.
60-MHz-¹H-NMR (D₂O): d(ppm) = 1.8 (m, 4H, CH₂-Arginin), 2.15 (s, 3H, CH₃-Acesulfam), 3.25 (t, J_{CH2,CH} = 5 Hz, 2H, CH₂-Arginin), 3.8 (t, J_{CH,CH2} = 5 Hz, 1H, CH-Arginin), 5.7 (s, 1H, CH-Acesulfam)

### Beispiel 2

### Herstellung des 1:2-L-Arginin-Acesulfam-Adduktes

In 20 ml Wasser werden 15 mmol (2,613 g) L-Arginin und 30 mmol (4,894 g) Acesulfam-H gelöst. Das Reaktionsgemisch wird anschließend im Vakuum eingeengt. Es resultieren mit 100 % Ausbeute farblose Kristalle, die laut ¹H-NMR als 1:2-Addukt vorliegen.
60-MHz-¹H-NMR (D₂O): d(ppm) = 1.8 (m, 4H, CH₂-Arginin), 2.15 (s, 6H, CH₃-Acesulfam), 3.25 (t, J_{CH2,CH} = 5 Hz, 2H, CH₂-Arginin), 4.1 (t, J_{CH,CH2} = 5 Hz, 1H, CH-Arginin), 5.7 (s, 2H, CH-Acesulfam)

### Beispiel 3

### Herstellung des 1:1:1-L-Arginin-Acesulfam-Saccharin-Adduktes

In 20 ml Wasser werden 15 mmol (2,613 g) L-Arginin und 15 mmol (2,447 g) Acesulfam-H gelöst. Anschließend werden 15 mmol (2,748 g) Saccharin-H hinzugefügt. Das Reaktionsgemisch wird anschließend im Vakuum eingeengt. Es resultieren mit 100 % Ausbeute farblose Kristalle, die laut ¹H-NMR als 1:1:1-Addukt vorliegen.
60-MHz-¹H-NMR (D₂O): d(ppm) = 1.8 (m, 4H, CH₂-Arginin), 2.2 (s, 3H, CH₃-Acesulfam), 3.35 (t, J_{CH2,CH} = 5 Hz, 2H, CH₂-Arginin), 4.1 (t, J_{CH,CH2} = 5 Hz, 1H, CH-Arginin), 5.85 (s, 1H, CH-Acesulfam), 8.1 (s, 4H, H-Saccharin)

## Patentansprüche

1. Salz aus einer basisch reagierenden Aminosäure mit mindestens einem sauer reagierenden Süßstoff, wobei Aminosäure und Süßstoff im molekularen Verhältnis 1:1 oder 1:2 vorliegen und wobei der Süßstoff ausgewählt wird aus einem oder mehreren der folgenden Süßstoffe: Acesulfam, Aspartam, Alitam, Cyclamat, Glycyrrhizin, Neotam, Saccharin und Gluconsäure bzw. Gluconat.

2. Salz gemäß Anspruch 1, **dadurch gekennzeichnet, dass** als Aminosäure Arginin, Lysin, Histidin, Tryptophan oder Ornithin eingesetzt wird.

3. Salz gemäß Anspruch 1 oder 2, **dadurch gekennzeichnet, dass** zwei verschiedene Süßstoffe im Molekül enthalten sind.

4. Verfahren zur Herstellung einer Verbindung nach einem oder mehreren der Ansprüche 1 bis 3, wobei man entweder
1) die Aminosäure mit einem oder 2 gleichen oder verschiedenen Süßstoffen, in Form ihrer freien Säure umsetzt und das gebildete Reaktionsprodukt isoliert, oder
2) die Aminosäure mit einem oder 2 gleichen oder verschiedenen Süßstoffen, in Form ihrer physiologisch verträglichen Salze in Gegenwart einer physiologisch verträglichen Säure umsetzt und das gebildete Reaktionsprodukt isoliert.

5. Verfahren nach Anspruch 4, **dadurch gekennzeichnet, dass** die Reaktion in Gegenwart von Wasser und/oder mit Wasser mischbare Lösungsmittel als Lösungsmittel durchgeführt wird.

6. Verfahren nach den Ansprüchen 4 oder 5, **dadurch gekennzeichnet, dass** die physiologisch verträgliche Säure z.B. Salzsäure, Schwefelsäure, Phosphorsäure oder Essigsäure ist.

7. Verwendung der Salze gemäß Anspruch 1 als Aminosäurequellen in Lebensmitteln, Nahrungsergänzungsmitteln und Arzneimitteln.

## Claims

1. A salt of a basic-reacting amino acid with at least one acidic-reacting sweetener, wherein amino acid and sweetener are present in a molecular ratio of 1:1 or 1:2 and wherein the sweetener is selected from one or more of the following sweeteners: acesulfame, aspartame, alitame, cyclamate, glycyrrhizin, neotame, saccharin and gluconic acid or gluconate.

2. A salt as claimed in claim 1, wherein the amino acid used is arginine, lysine, histidine, tryptophan or ornithine.

3. A salt as claimed in claim 1 or 2, wherein two different sweeteners are present in the molecule.

4. A process for preparing a compound as claimed in one or more of claims 1 to 3, which comprises either
1) reacting the amino acid in the form of its free acid with one or two identical or different sweeteners and isolating the reaction product formed, or
2) reacting the amino acid with one or two identical or different sweeteners in the form of their physiologically compatible salts in the presence of a physiologically compatible acid and isolating the reaction product formed.

5. The process as claimed in claim 4, wherein the reaction is carried out in the presence of water and/or with water-miscible solvent as solvent.

6. The process as claimed in either claim 4 or 5, wherein the physiologically compatible acid is, for example, hydrochloric acid, sulfuric acid, phosphoric acid or acetic acid.

7. The use of a salt as claimed in claim 1 as amino acid source in foods, food supplements and drugs.

## Revendications

1. Sel d'un acide aminé à réaction basique avec au moins un édulcorant à réaction acide, dans lequel acide aminé et édulcorant sont présents en un rapport molaire de 1:1 ou 1:2 et dans lequel l'édulcorant est choisi parmi un ou plusieurs des édulcorants suivants : acésulfame, aspartame, alitame, cyclamate, glycyrrhizine, néotame, saccharine et acide gluconique ou gluconate.

2. Sel selon la revendication 1, **caractérisé en ce qu'**on utilise comme acide aminé l'arginine, la lysine, l'histidine, le tryptophane ou l'ornithine.

3. Sel selon la revendication 1 ou 2, **caractérisé en ce que** deux édulcorants différents sont contenus dans la molécule.

4. Procédé pour la préparation d'un composé selon une ou plusieurs des revendications 1 à 3, dans lequel soit
1) on fait réagir l'acide aminé avec un ou deux édulcorants identiques ou différents, sous forme de leur acide libre, et on isole le produit de réaction formé, soit
2) on fait réagir l'acide aminé avec un ou deux édulcorants identiques ou différents, sous forme de leurs sels physiologiquement acceptables, en présence d'un acide physiologiquement acceptable, et on isole le produit de réaction formé.

5. Procédé selon la revendication 4, **caractérisé en ce que** la réaction est effectuée en présence, en tant que solvant, d'eau et/ou de solvants miscibles à l'eau.

6. Procédé selon la revendication 4 ou 5, **caractérisé en ce que** l'acide physiologiquement acceptable est par exemple l'acide chlorhydrique, sulfurique, phosphorique ou acétique.

7. Utilisation des sels selon la revendication 1, en tant que sources d'acides aminés dans des produits alimentaires, des compléments alimentaires et des médicaments.
